# EUROPEAN PATENT APPLICATION

(11) **EP 2 133 118 A2**
(43) Date of publication of application: **16.12.2009**
(21) Application number: 09162583.0
(22) Date of filing: 12.06.2009
(51) Int. Cl.: A61N 2/08

(54) **Magneto therapeutic device and intermediate layer**

(30) Priority: 13.06.2008 NL 1035583
(71) Applicant: Pegasus slaapcomfort V.O.F., 5701 NJ Helmond (NL)
(72) Inventor: Van Lier, Johannes Jacobus Cornelia, 5591 BE, Heeze (NL); Severens, Joannes Arnoldus Leonard, 5721 AL, Asten (NL)
(74) Representative: Jilderda, Anne Ayolt

(57) **Abstract**

A magneto therapeutic device comprises a therapeutic mattress with a bottom layer and a top layer and an intermediate layer comprising magnets and natural crystal enclosed between the bottom layer and top layer. The magnets are arranged in the intermediate layer in a polygonal shape and the natural crystal is positioned in a centre of the polygonal shape. A head part of the mattress where the head is situated comprises only crystals. The specific shape in which the magnets and crystal are arranged in the mattress and a specific orientation of the poles of the magnets improves the therapeutic properties of the device.

## Description

The present invention relates to a magneto therapeutic device comprising a therapeutic mattress with a bottom layer and a top layer and an intermediate layer comprising magnets and natural crystal enclosed between the bottom layer and the top layer.

Therapeutic mattresses are widely used by humans to promote health, reduce stress and radiation damage, change polarity of the human body and enhance blood circulation. Known therapeutic mattresses comprise a series of magnets to apply magnetic therapy to the user, in which the users body is exposed to magnetic fields to provide therapeutic and restorative treatment to the body. The effect of magnetic therapy, in which magnets are brought into close proximity with the body, is based on Faraday's Law of Magnetic Induction as well as the Hall Effect which charged particles experience. Hence the principle is based on a force acting on them when the charged particles move through a magnetic field in a perpendicular direction. In particular human blood comprises many charged molecules and particles such as electrolytes and various types of ions. These charged molecules and particles experience a force, including an aligning force, when moving through a magnetic field. As a result the molecules and particles generate heat while travelling through the blood vessel. This causes the blood vessel to widen so that blood circulation is promoted.

Importantly, the human body consists of approximately 70 percent water. Polar molecules such as water respond in a similar manner as ions to magnetic fields. Restorative effects might arise through the alignment of water molecules as they pass through the magnetic field. The water molecules rotate to align themselves with the field. Such alignments would alternate with the magnetic polarity as these molecules travel through different regions of such magnetic polarity. The motion of the rotating water molecules might also cause heating together with the ions thus increasing blood circulation. Also the motion of the water molecules might cause mixing of the blood at the molecular level. Hence the blood and/or immune system may be able to more readily address foreign matter which would enhance the vitality of the body. More theories and possibilities are still under investigation.

Moreover, research stresses the possible damage on a physical body caused by wireless technology devices such as GSM's and WIFI. Particularly in the last decade there has been a considerable increase in several forms of radiation caused by electrical power plants, magnetrons, TV and GSM antenna's etc., exposure to which causes changes in polarity in the human body which affects a body balance, induces stress and eventually causes several types of illnesses. It is known that magneto therapeutic treatment of the human body reduces stress, restores the polarity and hence improves health.

Several published patents describe various designs for the structure of magnets of different polarity to generate spatially diverse magnetic fields like Latzke (U.S. Pat. No. 4,489,711 issued Dec.25, 1984) or Ardizzone (U.S. Pat. No. 5,277,692 issued Jan. 11, 1994; U.S. Pat. No. 5,514,072 issued May 7, 1996 and U.S. Pat. No. 5,538,495 issued Jul. 23, 1996).

Besides magnets, the metaphysical properties of crystals with respect to improving the health of a human body are also well known. For example, natural clear quartz has proven beneficial in restoring polarity in the human body and hence reduces stress and improves vitality. Several studies have been published considering the effect of radiation on the infrared absorption of quartz crystals such as Sibley, W.A.;Martin, J.J.; Wintersgill, M.C.; Brown, J.D., Journal of Applied Phys., vol. 50, issue 8, pp. 5449-5452 (1979). However, there are also other beneficial types of crystals with respect to enhancing the health of a physical body.

Also the use of such materials as magnets and natural crystals in combination for improving human health is well known. For example the use of a combination of magnets and natural jade in a health-care water bed mattress is known from Chinese Pat. No. CN2009/41944 issued Sept. 5, 200. The materials radiate far infrared rays or anions, have antibacterial properties, and promote blood circulation and metabolism. Unlike cations the generated anions, as a result of the use of magnets and crystals, accelerate ionization of mineral components in blood to alkalify blood and thus purify blood and promote electrical exchange of materials in cell membranes. Moreover the anions increase an amount of gamma-globulins in the body to enhance the immune system. Cations secrete an excessive amount of serotonin and histamine in the human body which negatively affects health and deteriorates the ability of aerobic respiration.

Usually people wear magnets or crystals in the form of a necklace or bracelet. However, magnets and crystals have not yet been arranged together in specific polygonal shapes to improve health.

It is of advantage to implement magneto therapy together with crystal therapy in mattresses. As such, mild but deep treatment could then be affected for a period of several hours when a user rests or sleeps on the mattress. The users daily and ongoing activities is therefore not interfered with.

Hence a purpose of the present invention is to develop a therapeutic mattress in which a layer of specifically oriented magnets and natural crystals is incorporated.

Another purpose of the invention is to develop optimal structural combinations or shapes of magnets and natural crystals in order to improve physical health and also to determine which types of magnets and crystals are most suited. At the same time the economic aspects with respect to technically produce this product has to be considered, e.g. an optimal combination between high performance and a reasonable cost price. Also a possibility to recover the magnets and crystals when such a mattress is recycled has to be considered.

Another problem to be solved is the field strength of the magnets and the distance from the top layer of the mattress where the magnets and crystals are implemented in the intermediate layer in order to generate an optimal field strength at a surface of the mattress to improve the health of the human body.

Furthermore the optimal orientation of the dipole magnets has to be considered together with the optimal positions of the crystals.

Another problem to be solved is which coating material is most suited for the magnets without interfering with the magnetic field parameters in order to prohibit oxidation as a result of contact with respiratory fluids while a person rests or sleeps on the mattress.

Also a solution has to be found to fix the magnets and crystals in their positions without affecting a flexibility of the mattress, in order not to interfere with the sleeping qualities of the mattress in which the intermediate layer with magnets and crystals is incorporated.

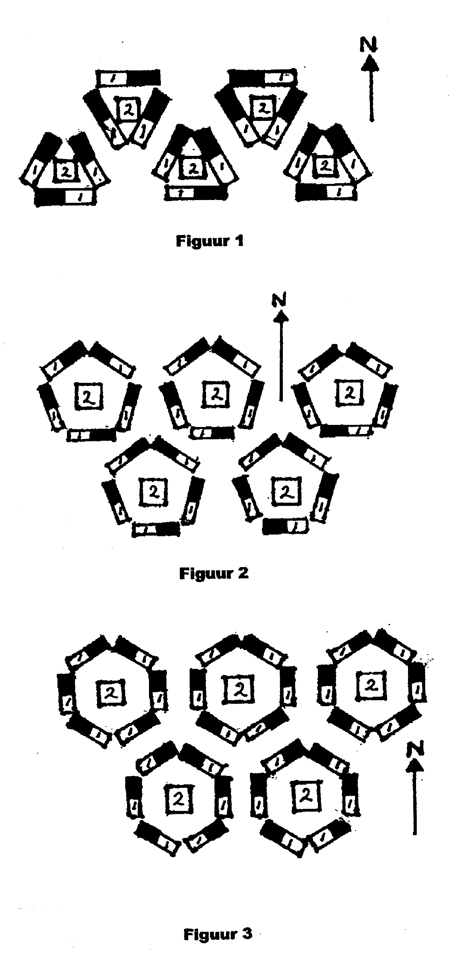

In order to achieve the intended objects a magneto therapeutic device of the type stated in the preamble has the feature according to the present invention that the magnets are arranged in the intermediate layer in a polygonal shape and the natural crystal is positioned in a centre of the polygonal shape. This specific polygonal shape of magnets and the crystal in the centre of each polygonal shape radiates large amounts of anions and far-infrared rays and has been invented to reduce stress in the physical body, enhance healthy metabolism, promote blood circulation, reduce blood pressure and reduce stiffness of the body. In addition, this special arrangement of dipole magnets and crystals offers protection against the daily exposure of the body to various forms of wireless radiation which causes damage to living cells in the body and leads to a reduced vitality. Moreover, the typical arrangement of magnets and crystals forms a special field. It can achieve the effect of dredging venations, getting rid of extra fat in the body and slow down the aging of skin. Examples of possible regular polygonal shapes are shown in figures 1, 2 and 3.

The field strength (flux density) of the magnets has to be considered in order not to exceed 3 Gauss at the surface of the mattress on the top layer side. Research has shown that living cells are negatively influenced in their cell metabolism and multiplication process, i.e. mitosis, if the magnetic field strength exceeds the value of 3 Gauss. Typically the magnetic field strength of our earth at the surface is in the order of 1 Gauss. Therefore a preferred embodiment of the magneto therapeutic device according to the invention is **characterized in that** a strength of a magnetic field of the magnets at a surface of the mattress at a top layer side ranges between 1 to 3 Gauss. It has been found that such a strength of the combined magnetic field at the surface of the mattress gives optimal results.

In a further preferred embodiment the magneto therapeutic device according to the invention is **characterized in that** a distance of the magnets to the surface of the mattress is in a range from 1 to 12 cm and is preferably 3 to 5 cm. Depending on the field strength of the used magnets and the type of mattress used, the specific layer of magnets and crystals has to be implemented at a certain distance from the surface of the mattress in order to obtain the optimal field strength at the surface of the mattress and thus the best magneto therapeutic results. It was found that freely available, and therefore relatively cheap, common magnets give very good results when applied in the intermediate layer at a distance of 1 to 12 cm from the surface of common mattresses. However, more preferably the magnets are applied at a distance of 3 to 5 cm from the surface, because at such a distance the comfort of the mattress as experienced by a user is unaltered, e.g. the user will not notice the presence of the magnets and crystal in the mattress.

Considering the magnetic properties of the magnets various types and forms of dipole magnets can be used as long as they are corrosion resistant and have a specific strength. As an example, in case of a 3 cm distance of the magnet/crystal layer from the surface of the mattress, we obtained good results with anisotropic ferrite magnets with an anti-corrosive nickel coating and a Max. energy production (BH)MAX ranging from 3.4- 4.5 MGOe. In this case the field strength at the surface of the mattress varied between 1 and 2 Gauss. The shape of the said dipole magnetic pieces and natural crystal pieces can be square, rectangular or round. As an example, a typical measure of the crystal plates we used in this example is 20x20x3 mm (L,B,W) and a typical measure of the magnets we used is 25x19x4,9 mm (L, B, W). In a further preferred embodiment the magneto therapeutic device according to the present invention is **characterized in that** the magnets comprise anisotropic magnets and preferably comprise ferro-strontium magnets.

In a further preferred embodiment the magneto therapeutic device according to the present invention is **characterized in that** the magnets are arranged in a specific orientation with their poles. Arranging the magnets with their poles in a typical orientation has been found to lead to good results in enhancing the health of the physical body. In a particular embodiment the magneto therapeutic device according to the present invention is **characterized in that** the magnets with their poles are arranged in a preferred earthmagnetic field orientation. It is most beneficial for the health when the mattress is aligned as much as possible with the magnetic field lines of the earth i.e. a head part of the mattress points to the magnetic north and a body part points to the magnetic south.

In a further particular embodiment the magneto therapeutic device according to the present invention is **characterized in that** the mattress comprises visual indicator means to give an indication of an orientation of the mattress. Such indicator means are helpful for a user to position the mattress in an optimal orientation. For example the indicator means could give an indication of which side of the mattress has to be pointed to the magnetic north pole of the earth.

In a further preferred embodiment the magneto therapeutic device according to the present invention is **characterized in that** the magnets and crystal are attached to at least one sheet of woven fabrics in the intermediate layer, and in a particular embodiment the magneto therapeutic device according to the present invention is **characterized in that** the intermediate layer comprises at least two sheets of woven fabric and the magnets and crystal are attached between said sheets. The magnets and crystals can be attached in various ways e.g. sewed or glued or taped etc. to a sheet made of woven fabrics in the intermediate layer. In this way the magnets and crystals can be fixed in their specific positions while the mattress still exhibits a large flexibility which enhances the sleeping comfort. In particular by enclosing the magnets and crystals between two sheets of woven fabric, the layer of magnets and crystals can be easily retrieved and used again in a recycling process of the mattress. This reduces production costs and is also beneficial for the environment.

In a further preferred embodiment the magneto therapeutic device according to the present invention is **characterized in that** the mattress comprises a head part and a body part, and at least the body part comprises the magnets and crystal in the intermediate layer. The human head is more sensitive for exposure to magnetic fields of 1-3 Gauss for a period up to a full night sleep in comparison with the rest of the human body. Therefore, in a further particular embodiment the magneto therapeutic device according to the present invention is **characterized in that** the head part comprises only magnets or only crystal or a combination of magnets and crystal in the intermediate layer. Thus it is possible to use a different arrangement of magnets, crystals and a combination of magnets and crystals for the head part of the mattress where the head of a user is situated compared to the body part where the body of a user is situated during use. For instance only crystals in a polygonal arrangement in the head part of the mattress can be used, or in a similar way only magnets can be used or a combination of magnets and crystals can be used.

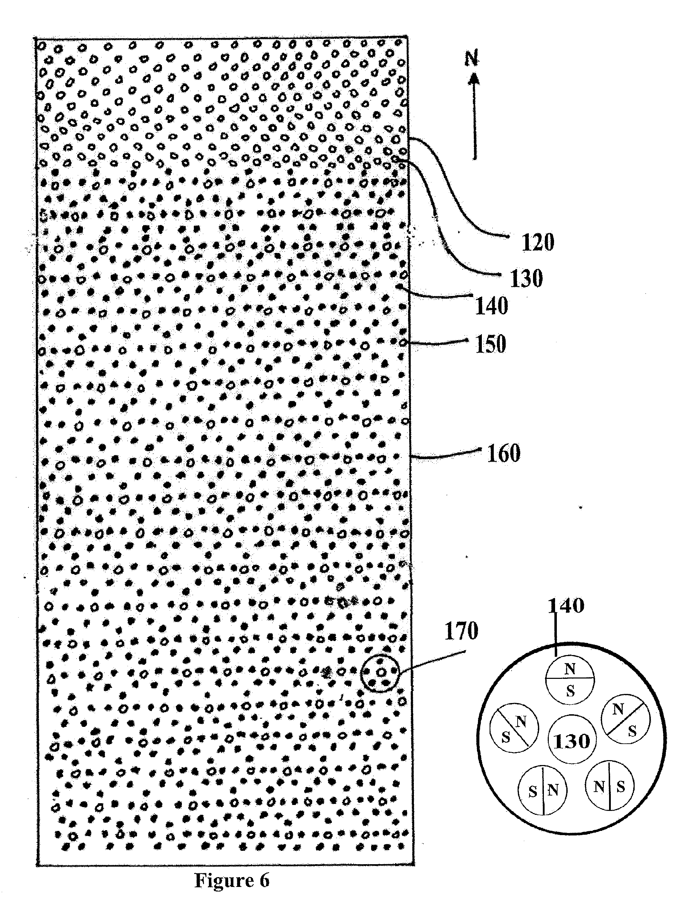

In a further preferred embodiment the magneto therapeutic device according to the invention is **characterized in that** a maximum dimension of the magnets is in a range from 0.5 cm to 15 cm, preferably in a range from 1 cm to 6 cm, and more preferably in a range from 2 to 4 cm. Considering the dipole interactions of the magnets, the distance of the magnets from each other in the shape can differ between 1,5 and 6 cm depending on the strength and size of the magnets. As an example the most effective orientation of the poles of the various magnets in the shape is shown in three examples of a regular multi-cornered (polygonal) arrangement. In these examples the distance between the magnets is 1,8 cm. In case of a triangular arrangement (Figure 1) of the magnets (size 25x19x4,9 mm, LxBxW) with a distance of 1,8 cm from each other the typical average number of magnets per m2 is 550 and the typical average number of crystal plates (size 20x20x3 mm, LxBxW) is 165 per m2. In case of pentagonal structures with similar characteristics (Figure 2) the typical average number of magnets per m2 is 450 and the typical average number of crystal plates per m2 is 112. In case of a hexagonal structure with similar characteristics and sizes (Figure 3) the typical average number of magnets per m2 is 432 and the typical average number of crystal plates per m2 is 84. As an example, in the head part of the mattress where the head is situated, in case only crystal plates with size 20x20x3 mm (LxBxW) are implemented in a triangular structure, the number of crystal plates in this particular part typically amounts to 365 plates per m2 (Figure 6).

Any type of natural crystal can be used as long as the crystal is not solvable in water. The metaphysical properties of crystals with respect to improving the health of a human body are well known. Various natural types of crystals, such as rock crystal, can be used according to their physical and metaphysical properties to enhance the health of a human body also depending on which type of equilibrium has to be restored. Natural clear quartz has proven to have beneficial properties in restoring polarity in the human body and hence reduce stress and improve vitality. However, other beneficial types of crystal like amethyst, citrine quartz, jade, red jasper, hematite and aquamarine with respect to enhancing the general health of a physical body may be used. Preferably natural clear quartz crystal is used because of its excellent properties to restore the health. Natural clear quartz radiates in the far infra-red and has excellent metaphysical qualities to improve health and vitality of a human body.

The anisotropic magnets can be arranged in any type of regular or irregular multi-cornered structures. For instance triangular arrangements, pentagonal arrangements and hexagonal arrangements of the anisotropic magnets can be used and lead to good health results. Examples of these arrangements and their specific orientation of the magnetic poles are given in figure 1, 2 and 3.

The present invention further relates to an intermediate layer as used in the magneto therapeutic device of the present invention.

In a preferred embodiment the intermediate layer according to the present invention comprises at least one sheet of fabric, and preferably comprises an antibacterial knitted or woven sheet of fabric.

The invention will be further elucidated hereinbelow on the basis of exemplary embodiments of a magneto therapeutic device according to the invention, shown in the drawings. Herein:
Fig. 1 shows a schematic drawing of a triangular orientation of the anisotropic magnets with their dipole positions and crystals in which the numbers 1 indicate the south poles of the magnets and the numbers 2 indicate the position of the crystal plates in the centre of each triangle. The earth magnetic north is given to show the desired alignment of the structure in the mattress with the magnetic north-south field lines of the earth.
Fig. 2 shows a schematic drawing of a pentagonal orientation of the anisotropic magnets with their dipole positions and crystals in which the numbers 1 indicate the south poles of the magnets and the numbers 2 indicate the position of the crystal plates in the centre of each pentagon . The earth magnetic north is given to show the desired alignment of the structure in the mattress with the magnetic field lines of the earth.
Fig. 3 shows a schematic drawing of a hexagonal orientation of the anisotropic magnets with their dipole positions and crystals in which the numbers 1 indicate the south poles of the magnets and the numbers 2 indicate the position of the crystal plates in the centre of each regular hexagon. The earth magnetic north is given to show the desired alignment of the structure in the mattress with the magnetic field lines of the earth.

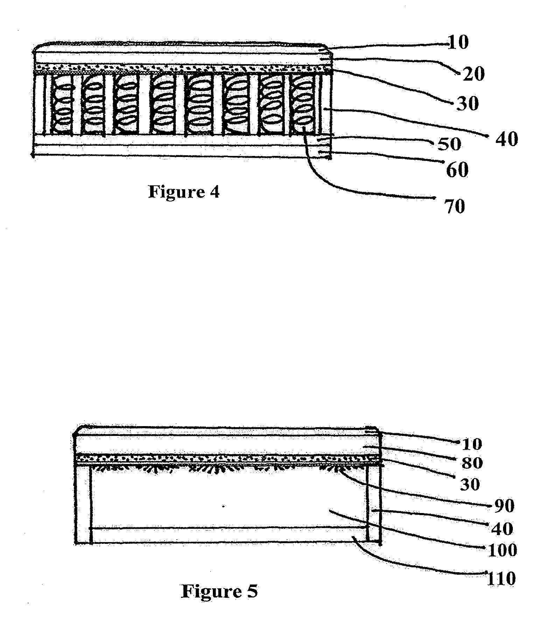

Fig. 4 shows a cross section of the various layers of a mattress with springs where the magnet/crystal layer is incorporated.
Fig. 5 shows a cross section of the various layers of a cold foam mattress where the magnet/crystal layer is incorporated.
Fig. 6 shows an example of the magnet/crystal structure for a common size 90x200 cm mattress in which the magnets are arranged in a pentagonal shape and the crystals in the head part are arranged in a triangular shape.

A more detailed description of figure 4, 5 and 6 is given below.

Figure 4 shows a cross section of a mattress used in combination with a box spring. The mattress consists of a top layer (10 and 20), an intermediate layer (30, 40 and 70) and a bottom layer (50 and 60). Layer 10 (thickness 2 cm) of the top layer can be surface treated with any health material. Layer 10 consists of wool, natural cotton or synthetic material. Layer 20 (2 to 3 cm) of the top layer consists of foam. Layer 30 of the intermediate layer comprises magnets and crystals which are fixedly attached to one sheet (approx. 1 cm thickness). Just above the springs the layer 30 consists of felt to balance out the pressure of the physical body sleeping on the mattress. The springs 70 have 4 or 5 windings. The layer 40 of the mattress consists of foam. The bottom layers 50 and 60 consist of different types of foam.

There is also the possibility to construct a symmetrical mattress with two layers of magnets/crystals or a layer of magnets/crystals approximately in the middle of the mattress. In this way the mattress can be used on both sides.

In figure 5 a cross section is shown for an example of a cold foam mattress with a top part (10 and 80), a middle part (30, 40, 90 and 100) and a bottom part (110).
Layer 10 (2 cm thickness) consists of wool, natural cotton or synthetic material and can be surface treated with health materials. Layer 80 consists of foam. Layer 30 (approx. 1 cm thickness) consists of magnets and crystals which are fixedly attached between two sheets. Layer 100 (16 cm thickness) consists of foam and has zones (90) to improve the quality of sleeping. The surrounding material 40 consists of foam. The bottom part 110 consists also of foam. The several layers consist of HR (High Resillience) cold foam (several types can be used with different qualities such as HR 35, HR 50, HR 55 or HR 60) of 16 cm as an example and on top a layer consisting of synthetic material and cotton. The position of the magnet/crystal layer is clearly shown. The magnet/crystal layer 30 comprises a pentagonal arrangement of anisotropic ferrite magnets with a nickel coating with natural clear quartz crystals in the centre of each pentagon. The specifications of the anisotropic ferrite magnets are: remanence Br approximately 4000 Gauss, Coercivity KOe: HcB 2800-3000 KA/M ; HcJ > 2900 KA/M, Max. energy production (BH)MAX 3.4-3.8 KJ/m3, size 25x19x4,9 mm (L,B,W). These magnets are used as an example of at least one way of carrying out the invention. The shape of the said dipole magnetic pieces and natural clear quartz pieces can be square, rectangular or round. In this example the typical measure of the crystal plates is 20x20x3 mm (L,B,W). In this example the layer of magnets and natural clear quartz crystals is implemented 3 cm under the top layer of the mattress to achieve the desired magnetic field strength at the surface of the mattress.

In figure 6 an example is shown of the positions of the magnets and the natural crystals in a therapeutic mattress with common size 90x200 cm according to the present invention. The head part 120 of the mattress in this example (30 cm x 90 cm) is free of magnets and incorporates only triangular structures of natural crystal 130. There exists a magnet and crystal free zone of 5 cm from the edges of the mattress. The preferable alignment to the magnetic north is indicated with an arrow. A detail of the specific orientation 170 shows the orientation of the poles of the round magnets 140. The layer 160 consists of any kind of woven fabrics suited to attach the magnets and crystals. The body part 150 of the mattress consists of a pentagonal arrangement of round dipole magnets 140 where everey centre of a pentagon, contains a natural crystal 130.

The magnets and crystals can be attached in any way e.g. by sewing, with glue, with tape or any other method. In this example the magnets and crystals are between two inner sheets of woven fabrics which are widely used in bedding. In this way the magnets and natural crystals, e.g. clear quartz, are fixed to their position and at the same time the layer is flexible in order not to interfere with other sleeping qualities of the mattress in which the layer is incorporated.

Although the invention has been elucidated in the foregoing on the basis of a single exemplary embodiment, it will be apparent that the invention is by no means limited thereto. On the contrary, many variations and embodiments are still possible within the scope of the invention.

## Claims

1. Magneto therapeutic device comprising a therapeutic mattress with a bottom layer and a top layer and an intermediate layer comprising magnets and natural crystal enclosed between the bottom layer and top layer, **characterized in that** the magnets are arranged in the intermediate layer in a polygonal shape and the natural crystal is positioned in a centre of the polygonal shape.

2. Magneto therapeutic device according to claim 1, **characterized in that** a strength of a magnetic field of the magnets at a surface of the mattress at a top layer side ranges between 1 to 3 Gauss.

3. Magneto therapeutic device according to claim 2, **characterized in that** a distance of the magnets to the surface of the mattress is in a range from 1 to 12 cm and is preferably 3 to 5 cm.

4. Magneto therapeutic device according to any one of the preceding claims, **characterized in that** the magnets are arranged in a specific orientation with their poles.

5. Magneto therapeutic device according to claim 4, **characterized in that** the magnets with their poles are arranged in a preferred earthmagnetic field orientation.

6. Magneto therapeutic device according to claim 4 or 5, **characterized in that** the mattress comprises visual indicator means to give an indication of an orientation of the mattress.

7. Magneto therapeutic device according to any one of the preceding claims, **characterized in that** the magnets and crystal are attached to at least one sheet of woven fabrics in the intermediate layer.

8. Magneto therapeutic device according to claim 7, **characterized in that** the intermediate layer comprises at least two sheets of woven fabric and the magnets and crystal are attached between said sheets.

9. Magneto therapeutic device according to any one of the preceding claims, **characterized in that** the mattress comprises a head part and a body part, and at least the body part comprises the magnets and crystal in the intermediate layer.

10. Magneto therapeutic device according to claim 9, **characterized in that** the head part comprises only magnets or only crystal or a combination of magnets and crystal in the intermediate layer.

11. Magneto therapeutic device according to any one of the preceding claims, **characterized in that** a maximum dimension of the magnets is in a range from 0.5 cm to 15 cm, preferably in a range from 1 cm to 6 cm, and more preferably in a range from 2 to 4 cm.

12. Magneto therapeutic device according to any one of the preceding claims, **characterized in that** the magnets comprise anisotropic magnets and preferably comprise ferro-strontium magnets.

13. Magneto therapeutic device according to any one of the preceding claims, **characterized in that** the natural crystal is a rock crystal.

14. Intermediate layer as used in the magneto therapeutic device according to any one of the preceding claims.

15. Intermediate layer according to claim 14 comprising at least one sheet of fabric, and preferably comprises an antibacterial knitted or woven sheet of fabric.
